(19) Europäisches Patentamt
European Patent Office
Office européen des brevets

(11) Veröffentlichungsnummer : **0 064 158**
**B1**

(12) # EUROPÄISCHE PATENTSCHRIFT

(45) Veröffentlichungstag der Patentschrift : **13.03.85**

(21) Anmeldenummer : **82102699.4**

(22) Anmeldetag : **31.03.82**

(51) Int. Cl.⁴ : **C 07 C121/75**, C 07 C121/66, A 61 K 31/275, C 07 C149/42, C 07 D317/60, C 07 D319/08, A 61 K 31/335

(54) **Omega-Cyan-1, omega-diphenyl-azaalkan-derivate, ihre Herstellung und diese enthaltende Arzneimittel.**

(30) Priorität : **10.04.81 DE 3114497**
**06.11.81 DE 3144150**

(43) Veröffentlichungstag der Anmeldung : **10.11.82 Patentblatt 82/45**

(45) Bekanntmachung des Hinweises auf die Patenterteilung : **13.03.85 Patentblatt 85/11**

(84) Benannte Vertragsstaaten : **AT BE CH DE FR IT LI LU NL SE**

(56) Entgegenhaltungen :
**EP-A- 0 029 175**
**DE-A- 1 643 429**

(73) Patentinhaber : **BASF Aktiengesellschaft**
**Carl-Bosch-Strasse 38**
**D-6700 Ludwigshafen (DE)**

(72) Erfinder : **Ehrmann, Oskar, Dr.**
**Reiterweg 19A**
**D-6800 Mannheim-Neuhermsheim (DE)**
Erfinder : **Raschack, Manfred, Dr.**
**Donnersbergstrasse 7**
**D-6714 Weisenheim am Sand (DE)**
Erfinder : **Gries, Josef, Dr.**
**Roemerweg 43**
**D-6706 Wachenheim (DE)**
Erfinder : **Kretzschmar, Rolf, Dr.**
**Oberer Bergelweg 4**
**D-6718 Gruenstadt (DE)**
Erfinder : **Lehmann, Hans Dieter, Dr.**
**Im Hefen 15**
**D-6945 Hirschberg (DE)**
Erfinder : **Friedrich, Ludwig, Dr.**
**Nibelungenstrasse 8A**
**D-6831 Bruehl (DE)**
Erfinder : **Wuppermann, Dirk, Dr.**
**Hinter den Ruestern 28**
**D-6713 Freinsheim (DE)**
Erfinder : **Zimmermann, Frank, Dr.**
**Am Wiesbrunnen 25**
**D-6730 Neustadt (DE)**
Erfinder : **Seitz, Werner, Dr.**
**Bismarckstrasse 22B**
**D-6831 Plankstadt (DE)**
Erfinder : **Treiber, Hans Joerg, Dr.**
**Sperberweg 1**
**D-6831 Bruehl (DE)**

**EP 0 064 158 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Erfinder : **Dengel, Ferdinand, Dr.**
**Am Hirschwald 15**
**D-6901 Wilhelmsfeld (DE)**
Erfinder : **Frank, Wolfram, Dr.**
**Bergstrasse 162**
**D-6900 Heidelberg (DE)**
Erfinder : **Kurbjuweit, Hans-Georg, Dr.**
**Talstrasse 33**
**D-6941 Weinheim-Hohensachsen (DE)**
Erfinder : **Mueller, Claus D., Dr.**
**Odenwaldring 84**
**D-6806 Viernheim (DE)**

**Beschreibung**

Die vorliegende Erfindung betrifft neue omega-Cyan-1, omega-diphenylazaalkan-derivate, deren Herstellung sowie A.zneimittel, welche diese Substanzen enthalten.

Es sind bereits eine Reihe von 7-Cyan-1,7-diphenyl-3-azaalkan-derivate bekannt (vgl. DE-PS 11 54 810). Als wirksamste dieser Verbindungen haben sich bislang das Verapamil (1,7-Bis-(3,4-dimethoxyphenyl)-3-methylaza-7-cyan-8-methyl-nonan) und das Gallopamil (1-(3,4-Dimethoxyphenyl)-3-methylaza-7-cyan-7-(3,4,5-trimethoxyphenyl)-8-methyl-nonan) erwiesen.

Es wurden nun neue Verbindungen gefunden, die dem Verapamil und Gallopamil überlegen sind.

Gegenstand der Erfindung sind omega-Cyan-1, omega-diphenylazaalkan-derivate der Formel I,

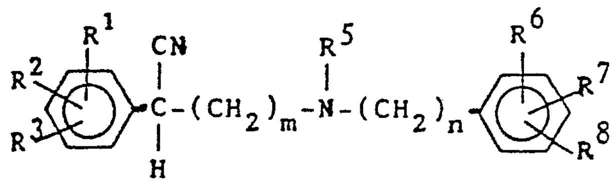

(I)

worin

$R^1$, $R^2$, $R^3$, $R^6$, $R^7$, $R^8$ gleich oder verschieden sind und Wasserstoffatome, Halogenatome, Hydroxylgruppen, Trifluormethylgruppen, $C_1$-$C_4$-Alkylgruppen, Nitrogruppen, $C_1$-$C_4$-Alkoxygruppen oder $C_1$-$C_4$-Alkylmercaptogruppen bedeuten, wobei auch jeweils zwei Reste in Nachbarstellung zusammen Methylendioxy-, Ethylendioxy- oder 1,3-Dioxatetramethylengruppen bilden können,

$R^4$ eine gerade oder verzweigte, gesättigte oder ungesättigte Alkylgruppe mit 9 bis 20 Kohlenstoffatomen ist,

$R^5$ ein Wasserstoffatom oder einen $C_1$-$C_4$-Alkylrest darstellt und

m und n gleich oder verschieden sind und die Zahlen 2 bis 4 bedeuten,

sowie deren Salze mit physiologisch verträglichen Säuren.

Als physiologisch verträgliche Säuren kommen z. B. in Frage : Salzsäure, Schwefelsäure, Phosphorsäure, Essigsäure, Malonsäure, Bernsteinsäure, Fumarsäure, Maleinsäure, Zitronensäure, Weinsäure, Milchsäure, Amidosulfonsäure und Oxalsäure.

$R^1$ bis $R^3$ sowie $R^6$ bis $R^8$ stellen vorzugsweise Wasserstoffatome oder Alkoxyreste, insbesondere Methoxyreste dar, $R^4$ stellt vorzugsweise einen geradkettigen Alkylrest mit 9 bis 14, insbesondere 11 bis 13 Kohlenstoffatomen und $R^5$ vorzugsweise die Methylgruppe dar.

Die neuen Verbindungen besitzen ein asymmetrisches C-Atom. Sie können daher in Form ihrer Antipoden hergestellt werden, vgl. DE-PS 20 59 923 und DE-PS 20 59 985.

Die neuen Verbindungen werden hergestellt, indem man

a) Phenylacetonitrile der Formel II,

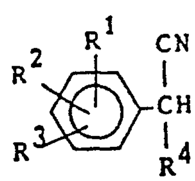

(II)

worin $R^1$ bis $R^4$ die angegebene Bedeutung haben, mit 1-Phenylazaalkanen der Formel III,

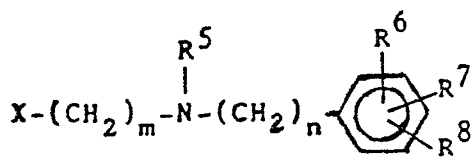

, (III)

worin $R^5$ bis $R^8$ sowie m und n die angegebene Bedeutung besitzen, und X eine Austrittsgruppe darstellt, umsetzt oder

b) omega-Cyan-1, omega-diphenyl-azaalkan-derivate der Formel IV,

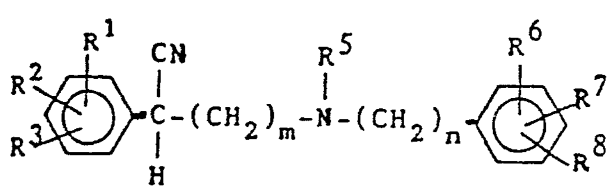

(IV)

worin $R^1$ bis $R^8$, m und n die angegebene Bedeutung haben, mit Verbindungen der Formel

$$R^4{-}Y \qquad (V)$$

worin $R^4$ die angegebene Bedeutung hat und Y eine Austrittsgruppe darstellt, zur Reaktion bringt oder

c) Phenylacetonitrile der Formel VI,

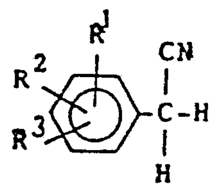

(VI)

worin $R^1$ bis $R^3$ die angegebene Bedeutung haben, mit 1-Phenylazaalkanen der Formel III und Verbindungen der Formel V umsetzt oder

d) Phenylacetonitrile der Formel VII,

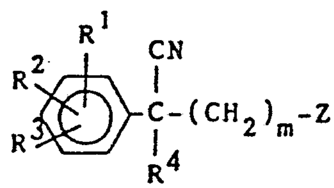

(VII)

worin $R^1$ bis $R^4$ und m die oben angegebenen Bedeutungen haben und Z eine Austrittsgruppe darstellt, mit einem Phenylalkylamin der Formel VIII,

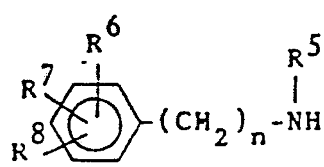

(VIII)

worin $R^5$ bis $R^8$ und n die obige Bedeutung besitzen, zur Reaktion bringt oder

e) omega-Amino-alkyl-phenylacetonitrile der Formel IX,

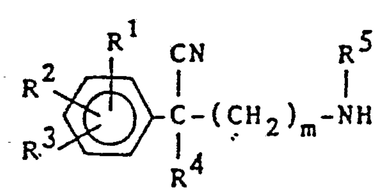

(IX)

worin $R^1$ bis $R^5$ und m die beschriebene Bedeutung haben, mit Phenylalkyl-derivaten der Formel X,

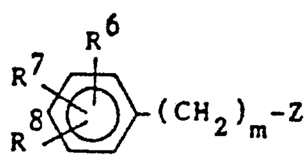

(X)

worin $R^6$ bis $R^8$, m und Z die oben angegebene Bedeutung haben, zur Reaktion bringt oder

f) omega-Cyan-1, omega-diphenyl-azaalkan-derivate der Formel XI,

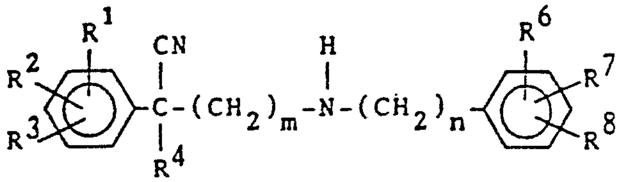

(XI)

worin $R^1$ bis $R^8$, m und n die angegebene Bedeutung haben, alkyliert oder

g) omega-Aminoalkyl-phenylacetonitrile der Formel IX mit Aldehyden der Formel XII,

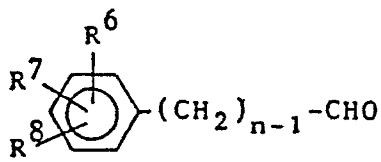

(XII)

worin $R^6$ bis $R^8$ und n dasselbe wie oben bedeutet, unter reduktiven Bedingungen umsetzt oder

h) Aldehyde der Formel XIII,

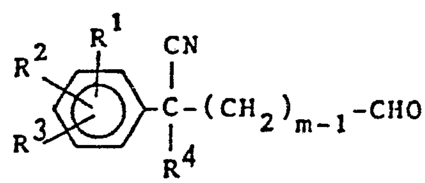

(XIII)

worin $R^1$ bis $R^4$ und m dasselbe wie oben bedeutet, mit Phenylalkylaminen der Formel VIII unter reduktiven Bedingungen umsetzt,

und die so erhaltenen Verbindungen, falls gewünscht und falls einer oder mehrere der Reste $R^1$, $R^2$, $R^3$, $R^6$, $R^7$ oder $R^8$ Alkoxygruppen sind, diese einer Etherspaltung unterwirft und/oder die so erhaltenen Verbindungen gegebenenfalls in ihre Salze mit physiologisch verträglichen Säuren überführt.

Die Umsetzung a) kann beispielsweise durchgeführt werden, indem man ein CH-acides Phenylacetonitril der Formel II in einem inerten Lösungsmittel mit einer Base metalliert und anschließend mit Verbindungen der Formel III umsetzt. Gegebenenfalls kann auch so verfahren werden, daß die Base zu einer Lösung von Verbindungen der Formel II und III zugegeben wird.

Als Basen kommen in Betracht: Alkalimetall-hydride, -hydroxide, -alkoholate, -amide und metallorganische Verbindungen. Vorzugsweise werden verwendet: Natriumamidpulver und -suspension, Kaliumhydroxidpulver, Butyllithium, Lithiumdiisopropylamid.

Als Lösungsmittel für die Reaktion eignen sich aromatische und aliphatische Kohlenwasserstoffe, jedoch sind auch höhersiedende aliphatische Ether und dipolar aprotische Lösungsmittel geeignet. Bevorzugt wird mit Toluol gearbeitet.

Reaktion a) kann auch nach einen phasentransferkatalysierten Verfahren durchführt werden. Als Katalysatoren finden quartäre Ammonium- und Phosphoniumsalze oder Kronenether Verwendung.

Die Reaktionstemperaturen sind von den eingesetzten Basen abhängig, z. B. wird mit Butyllithium bei Temperaturen zwischen 0° und − 100 °C und bei Verwendung von Natriumamid vorzugsweise bei 50° bis 150 °C gearbeitet.

Die Umsetzung von Verbindungen der Formel IV zu den erfindungsgemäßen Verbindungen (Verfahren b) erfolgt in ähnlicher Weise wie bei Verfahren a).

Als Reaktionspartner der Formel IV kommen beispielsweise in Frage: Alkanderivate mit einer Austrittsgruppe, wie Halogenide, Schwefelsäureester, Tosylate, Mesylate oder Triflate.

Bei Verfahren c) kann die Reihenfolge der Zugabe der Verbindungen III, V und VI beliebig gewählt und auf eine Isolierung von Zwischenprodukten verzichtet werden.

Die Reaktion d) erfolgt durch einfaches Erhitzen der Reaktionspartner auf vorzugsweise 120 bis 180 °C. Sie kann auch in einem Lösungsmittel erfolgen, doch ist dieses nicht erforderlich. Dasselbe gilt für die Umsetzung e). Als Z eignet sich in beiden Fällen Halogen, vorzugsweise Chlor und Brom.

Die Alkylierung f) gelingt mit Dialkylsulfaten oder Alkylhalogeniden in Gegenwart eines Säurefängers, wie Triethylamin oder Kaliumcarbonat. Zweckmäßigerweise arbeitet man in einem Lösungsmittel, wie Toluol oder in einem dipolar aprotischen Lösungsmittel, wie Dimethylformamid. Die Methylierung kann auch nach Leuckart-Wallach mit Formaldehyd/Ameisensäure durchgeführt werden.

Beim Verfahren g) und h) werden die beiden Reaktionspartner der Formel IX und XII bzw. XIII und VIII in einer Kondensation unter reduktiven Bedingungen umgesetzt.

Als Lösungsmittel sind aliphatische und aromatische Kohlenwasserstoffe, halogenierte Kohlenwasserstoffe, Ether, Alkohole oder niedrige Fettsäuren geeignet. Die Reaktionstemperaturen liegen zwischen 0 und 150 °C, vorzugsweise 20 bis 70 °C.

Als Reduktionsmittel kommen in Betracht : Wasserstoff in Gegenwart eines Katalysators, z. B. $PtO_2$, Pd/C, Nickel- oder Kobalt-Katalysatoren, nascierender Wasserstoff, den man aus Metall und Säure erhält, komplexe Metallhydride (z. B. $NaBH_4$) oder Hydriddonatoren (z. B. Ameisensäure).

Wird die Reduktion in Gegenwart eines Katalysators durchgeführt, so arbeitet man bevorzugt bei Atmosphärendruck.

Die Reaktionen a) bis h) sind beschrieben in DE-PS 11 54 810, DE-PS 11 58 083, DE-PS 20 59 923, DE-AS 22 63 527, DE-PS 26 31 222 und DE-OS 30 34 221.

Die bislang nicht beschriebenen Ausgangsstoffe der Formeln IV, VII, IX, X, XI und XIII lassen sich wie folgt herstellen :

Durch Umsetzen von Phenylacetonitrilen (VI) mit 1-Phenyl-omega-halogen-azaalkanen (vgl. III) in Gegenwart von Natriumamid in Toluol erhält man die Verbindungen IV.

Die Verbindungen VII lassen sich durch Umsetzen von 1-Cyan-1-phenyl-alkanen (II) mit Chlorpropanol und anschließende Reaktion des so erhaltenen Alkohols mit beispielsweise Thionylchlorid darstellen.

Die Verbindungen IX gewinnt man aus den Phenylacetonitrilderivaten VII durch Umsetzen mit Alkylaminen.

Die Substanzen der Formel X werden hergestellt, indem man z. B. die Phenylacetonitrile VI zu Phenylessigsäure hydrolysiert, letztere zum entsprechenden Alkohol reduziert und diesen beispielsweise mit Thionylchlorid chloriert.

Die Umsetzung von 1-Cyan-1-phenyl-alkanen (II) mit 1-Phenyl-omega-halogen-aza-alkanen (vgl. III) in Gegenwart von Natriumamid in Toluol führt zu den Verbindungen XI.

Durch Umsetzung von 1-Cyan-1-phenyl-alkanen (II) mit omega-Halogen-Aldehyddiethylacetalen in Gegenwart von Natriumamid in Toluol und anschließende Behandlung des so erhaltenen Reaktionsproduktes mit Säure gewinnt man die Verbindungen XIII.

Omega-Cyan-1, omega-diphenyl-azaalkanderivate der Formel I, welche durch Hydroxylgruppen substituiert sind, können aus den entsprechenden Alkoxyderivaten dadurch erhalten werden, daß man diese einer Etherspaltung unterwirft, die mit konzentrierter Bromwasserstoffsäure, Trifluoressigsäure, Bortrihalogeniden (-chlorid oder -bromid) oder mit Alkalimetallmercaptiden in dipolar-aprotischen Lösungsmitteln durchgeführt werden kann.

Die neuen Verbindungen sind geeignet zur Behandlung von schwerwiegenden funktionellen Erkrankungen des Herz-Kreislauf-Systems sowie von Kardiomyo- und Angiopathien. Sie wirken kardioprotektiv bei hypoxischen bzw. ischämischen Herzerkrankungen und bei nichtkoronarogenen Myokardschädigungen sowie bei Tachykardien und Rhythmusstörungen. Aufgrund ihrer antihypertensiven und antiaggregierenden Wirkkomponenten können sie auch zur Behandlung des hohen Blutdrucks und von Durchblutungsstörungen eingesetzt werden. Sie relaxieren die glatte Muskulatur und können daher zum Beispiel zur Lösung von Spasmen der Gefäße, der Bronchien, des Ureters und des Magen-Darm-Trakts sowie bei der Tokolyse verwendet werden. Ferner hemmen sie sekretorische Prozesse, die beispielsweise bei der Ulcusgenese (Magensäurefreisetzung) eine Rolle spielen, sowie allergische Reaktionen. Die neuen Verbindungen zeichnen sich durch eine gute orale Wirksamkeit und eine lange Wirkdauer aus.

Zum Nachweis der pharmakologischen Wirkungen wurden folgende Methoden verwendet :

1. Antihypertensive Wirkung

Die Substanzen wurden männlichen spontan hypertensiven Ratten (SHR) vom Okamoto-Stamm (Gewicht 300-400 g) oral appliziert. Der systolische Blutdruck wurde vor und 2, 6, 24 und gegebenenfalls 30 h nach der Applikation unblutig am Rattenschwanz mit Hilfe von Piezokristallaufnehmern ermittelt. Als ED 20 % wurde unter Berücksichtigung der Werte unbehandelter Kontrolltiere die Dosis bestimmt, welche den systolischen Druck um 20 % senkt.

Als Vergleichssubstanz diente Verapamil.

2. Antiarrhythmische Wirkung

Zur Bestimmung der antiarrhythmischen Wirksamkeit wurden die Substanzen Ratten (Stamm : Sprague-Dawley, Gewicht : 200-250 g) oral appliziert. 5 h später wurden die Tiere mit Thiobutabarbital-Natrium (100 mg/kg) i. p.) narkotisiert. Als arrhythmogene Substanz diente Aconitin, das 6 h nach Substanzapplikation i. v. infudiert wurde (Dosierungsgeschwindigkeit : 0.005 mg/kg.min). Bei nichtbehandelten Tieren traten nach 2,74 ± 0,07 min im EKG Arrhythmien auf, deren Eintritt durch Antiarrhythmika dosisabhängig verzögert werden kann. Bestimmt wurde die relative Verlängerung der Aconitininfusionsdauer (Δ %) durch die Prüfsubstanzen in der Dosierung von jeweils 46,4 mg/kg

Als Vergleichssubstanz diente Verapamil.

3. Kardioprotektive Wirkung gegen hypoxische Herzstoffwechselschäden

An narkotisierten Ratten (Stamm : Wistar, Gewicht : 250-350 g, Narkose : Thiobutabarbital 100 mg/kg i. p.) wurde durch standardisierte Beatmung mit einem Sauerstoffmangelgemisch (2 % $O_2$) eine drastische Verarmung des Myocards an energiereichen Phosphaten erzeugt. Die Bestimmung von Kreatinphosphat erfolgte unter Anwendung von Gefrierstoptechnik (flüssiger Stickstoff) in Muskelproben der Herzspitze nach der Methode von Lamprecht et al., 1974 (Lamprecht, W., P. Stein, F. Heinz, H. Weissner : Creatinphosphat in : Bergmeyer, H. U., Methoden der enzymatischen Analyse, Verlag Chemie, Weinheim, *2*, 1825-1829, (1974).

Die Applikation der Prüfsubstanzen erfolgte 6 h vor der Sauerstoffmangelbeatmung an die noch wachen Tiere. Bestimmt wurde der prozentuale Unterschied der Kreatinphosphat-Konzentration im Myocard bei mit Prüfsubstanz vorbehandelten Tieren im Vergleich zu unbehandelten Hypoxie-Kontrolltieren.

Als Vergleichssubstanz diente Verapamil.

4. Thrombozytenaggregationshemmende Wirkung

Die Substanzen wurden männlichen Sprague-Dawley-Ratten (200-250 g) oral appliziert. 1 h nach der Applikation wurde in Ether-Narkose Blut entnommen und durch Zentrifugation (300 g, 10 min Dauer bei 4 °C) thrombozytenreiches Plasma gewonnen. Die photometrische Messung der Thrombozytenaggregation erfolgte unter Zusatz von $MgCl_2$ (Endkonzentration 10 mmol/l) und von Collagen Stago (Endkonzentration 0,02 mg/ml) im Born-Aggregometer Mk3. Als Aggregationsmaß fand die maximale Extinktionsänderung/sec Verwendung.

Als ED 33 % wurde die Dosis bestimmt, welche die durch Collagen induzierte Thrombozytenaggregation um 33 % hemmt.

Als Vergleichssubstanz diente Verapamil.

5. Antiallergische Wirkung

Zur Prüfung wurde das Modell der passiven cutanen Anaphylaxie (PCA) verwendet.

Narkotisierte männliche Ratten (100-140 g) wurden durch intradermale Injektion (rasierte Rückenhaut) von 0,1 ml eines Ovalbumin-Antiserums sensibilisiert. Nach einer Sensibilisierungsperiode von ca. 48 h erfolgte die Behandlung (orale Applikation) mit den Prüfsubstanzen. Nach unterschiedlichen Latenzzeiten (2, 6, 12 und 24 h) wurde eine Antigen/Evansblau-Lösung den Versuchstieren intravenös injiziert. Jeweils 30 min später wurden die Tiere getötet, die Rückenhaut abgezogen und auf der inneren Oberfläche die kreisförmige Blaufärbung gemessen.

Die antiallergische Wirkung wurde als relative Hemmung (Δ %) des Durchmessers der Farbflecke angegeben. Als Vergleichssubstanz diente Verapamil.

Tabelle 1

Antihypertensive Wirkung, spontan hypertone Ratte (SHR), Applikation : per os

| Substanz des Beispiels Nr. | Senkung des systolischen Blutdrucks ED 20 % (mg/kg) 1) | | | | |
|---|---|---|---|---|---|
| | 2 h | R.W.2) | 6 h | R.W.2) | 24 h |
| 3 | 24.9 | 0.93 | 14.1 | 1.46 | 18.7 |
| 19 | 7.5 | 3.09 | 19.8 | 1.04 | 46.4 |
| 20 | 3.7 | 6.27 | 6.6 | 3.12 | 12.5 |
| 25 | 6.8 | 3.41 | 10.2 | 2.02 | 46.4 |
| 49 | 10.3 | 2.25 | | | 58.3 |
| Verapamil | 23.2 | 1.00 | 20.6 | 1.00 | — |

1) Dosis, welche den systolischen Blutdruck um 20 % senkt.
2) R.W. = Relative Wirksamkeit ; Verapamil = 1.00.

(Siehe Tabelle 2 Seite 8 f.)

Tabelle 2

Antiarrhythmische Wirkung, Ratte, Dosis 46,4 mg/kg Applikation : per os

| Substanz des Beispiels Nr. | Verlängerung der Aconitin-infusionsdauer (△%) |
|---|---|
| 3 | 122 |
| 5 | 107 |
| 7 | 88 |
| 8 | 88 |
| 12 | 128 |
| 13 | 188 |
| 14 | 98 |
| 15 | 88 |
| 16 | 61 |
| 19 | 50 |
| 23 | 86 |
| 24 | 68 |
| 25 | 186 |
| 46 | 54 |
| 47 | 104 |
| 53 | 63 |
| 58 | 53 |
| 59 | 61 |
| 61 | 69 |
| 62 | 86 |
| 63 | 70 |
| 67 | 65 |
| 70 | 59 |
| 72 | 96 |
| 75 | 71 |
| Verapamil | 29 1) |

1) nicht signifikant

Tabelle 3

Kardioprotektive Wirkung 6 h nach oraler Applikation an Ratten

| Substanz des Beispiels Nr. | maximal wirksame Dosis mg/kg | Kreatinphosphat Konz. im Myokard Abw. v. Kontr. in % |
|---|---|---|
| 3 | 40 | + 77 |
| 5 | 20 | + 69 |
| 7 | 40 | + 49 |
| 8 | 40 | + 35 |
| 10 | 40 | + 35 |
| 16 | 40 | + 50 |
| 17 | 40 | + 51 |
| 19 | 2 | + 38 |
| 20 | 5 | + 38 |
| 67 | 40 | + 53 |
| 72 | 40 | + 38 |
| 75 | 20 | + 80 |
| Verapamil | 40 | + 9 1) |

1) nicht signifikant.

Tabelle 4

Thrombozytenaggregationshemmende Wirkung, 1 h nach oraler Zufuhr, Ratte

| Substanz des Beispiels Nr. | Aggregationshemmung ED 33 % (mg/kg) 1) |
|---|---|
| 3 | 9,6 |
| 6 | 49,0 |
| 70 | 119 |
| 75 | 90,2 |
| Verapamil | 2) |

1) Dosis, welche die durch Collagen ausgelöste Thrombozytenaggregation um 33 % hemmt.
2) Eine Dosis von 46,4 mg/kg ist ohne antiaggregatorische Wirkung.

Tabelle 5

Antiallergische Wirkung nach oraler Gabe von 21,5 mg/kg Prüfsubstanz PCA Ratte ; Untersuchung zur Wirkdauer

| Substanz des Beispiels Nr. | % Hemmung Latenzzeit in Stunden 2 | 6 | 12 | 24 |
|---|---|---|---|---|
| 3 | 15 | 40 | 83 | 68 |
| Verapamil | 53 | 50 | 33 | 31 |

Die erfindungsgemäßen Verbindungen zeichnen sich durch folgende Wirkungen aus :

1. Antihypertensive Wirkung

Die antihypertensive Wirkung an SH-Ratten ist bei den erfindungsgemäßen Verbindungen (Tabelle 1) im allgemeinen stärker ausgeprägt als bei Verapamil. Hinzu kommt eine deutliche Zunahme der Wirkungsdauer. Im Gegensatz zu Verapamil, das in der subletalen Dosis von 100 mg/kg nach 24 h unwirksam war, sind die übrigen Substanzen (insbesondere Beispiel 3 und 20) auch noch zu diesem Zeitpunkt antihypertensiv aktiv.

2. Antiarrhythmische Wirkung

Die in Tabelle 2 aufgeführten erfindungsgemäßen Verbindungen verlängern die Aconitininfusionsdauer um 50 % (Beispiel Nr. 19) bis 188 % (Beispiel 13).
Sie unterscheiden sich damit deutlich von Verapamil, das in der Dosis von 46,4 mg/kg keinen signifikaten Einfluß auf aconitininduzierte Arrhythmien bei Ratten hat.

3. Kardioprotektive Wirkung

Die in Tabelle 3 zusammengefaßten Beispiele zeigen, daß die erfindungsgemäßen Verbindungen in Dosen von 2-40 mg/kg p. o. eine deutliche kardioprotektive Wirkung aufweisen.
Besonders stark wirken die Beispiele 3, 5 und 75. Verapamil ist bis 40 mg/kg p. o. unter den gewählten Versuchsbedingungen ohne signifikante Schutzwirkung.

4. Thrombozytenaggregationshemmende Wirkung

Unter den neuen Verbindungen fallen die der Beispiele 3, 70 und 75 durch eine bei oraler Zufuhr an der Ratte nachweisbare Hemmwirkung an der durch Collagen induzierten Thrombozytenaggregation auf (Tabelle 4). Verapamil ist unter gleichen experimentellen Bedingungen bis zu maximal tolerablen Dosis von 46,4 mg/kg ohne Einfluß auf die Thrombozytenaggregation.

5. Antiallergische Wirkung

Die erfindungsgemäße Verbindung (Beispiel 3) ist nach oraler Gabe am Modell der passiven cutanen Anaphylaxie der Ratte langanhaltend antiallergisch wirksam (Tabelle 5). Die Überprüfung der Wirkdauer ergab, daß die Verbindung nach Latenzzeiten von 12 und 24 h wesentlich stärker wirksam ist als die Vergleichsverbindung Verapamil und sich damit durch eine vergleichsweise längere Wirkungsdauer auszeichnet.
Die neuen Verbindungen können in üblicher Weise oral oder parenteral verabfolgt werden.
Die Dosierung hängt vom Alter, Zustand und Gewicht des Patienten sowie von der Applikationsart ab. In der Regel beträgt die tägliche Wirstoffdosis zwischen etwa 0,1 und 10 mg/kg Körpergewicht bei oraler Gabe und zwischen 0,01 und 1,0 mg/kg Körpergewicht bei parenteraler Gabe. Im Normalfall werden tägliche Dosen von 1 bis 5 mg/kg oral und 0,05 bis 0,25 mg/kg parenteral angewendet.
Die neuen Verbindungen können in den gebräuchlichen galenischen Applikationsformen fest oder flüssig angewendet werden, z. B. als Tabletten, Filmtabletten, Kapseln, Pulver, Granulate, Dragees, Suppositorien, Lösungen oder Dosieraerosole. Diese werden in üblicher Weise hergestellt. Die Wirkstoffe können dabei mit den gebräuchlichen galenischen Hilfsmitteln, wie Tablettenbindern, Füllstoffen, Konservierungsmitteln, Tablettensprengmitteln, Fließregulierungsmitteln, Weichmachern, Netzmitteln, Dispergiermitteln, Emulgatoren, Lösungsmitteln, Retardierungsmitteln und/oder Antioxidantien verarbeitet werden (vgl. H. Sucker, P. Fuchs, P. Speiser : Pharmazeutische Technologie, Thieme-Verlag, Stuttgart). Die so erhaltenen Zubereitungen enthalten den Wirkstoff normalerweise in einer Menge von 0,1 und 99 Gew.-%.
Die Erfindung wird nachstehend anhand von Beispielen erläutert. Die Dünnschichtchromatographien wurden an DC-Fertigplatten mit Konzentrierungszone der Firma E. Merk mit Kiesegel 60 F 254 durchgeführt.

Beispiel 1

1,7-Bis-(3-ethoxyphenyl)-3-methylaza-7-cyan-nonadecan

In einem Dreihalskolben, der mit Rührer, Tropftrichter, Rückflußkühler und Thermometer versehen ist, wurden 20,4 g (0,06 Mol) 1-Cyan-1-(3)-ethoxyphenyl)-tridecan (hergestellt durch phasentransferkatalysierte Alkylierung von 3-Ethoxyphenylacetonitril mit 1-Bromdodecan) und 16,9 g (0,06 Mol) 1-Chlor-4-methylaza-6-(3-ethoxyphenyl)-hexan [hergestellt anlog Arzneim.-Forsch. *28* (II), 2048 (1978) aus N-Methyl-beta-(3-ethoxyphenyl)-ethylamin und 1-Brom-3-chlorpropan] in 100 ml trockenem Toluol

gelöst. Anschließend wurden bei 100 bis 110 °C unter Rühren innerhalb von 30 min 9,3 g (0,07 Mol) 30 %ige toluolische Natriumamidsuspension zugetropft und weitere 90 min unter Rückfluß nachgerührt.

Die erhaltene Reaktionslösung wurde in 200 ml Eiswasser gegeben, die Toluolphase abgetrennt und zweimal mit Wasser gewaschen. Die Toluollösung wurde mit der erforderlichen Menge Salzsäure versetzt, das Toluol unter vermindertem Druck abdestilliert und der verbleibende Rückstand aus Aceton umkristallisiert. Es wurden 29,7 g (82 %) des Hydrochlorids erhalten, Fp. 132-134 °C.

In analoger Weise wurden erhalten :

2. 1,7-Bis-(2-methoxyphenyl)-3-methylaza-7-cyan-nonadecan-hydrochlorid, Fp = 60 bis 69,5 °C.

3. 1,7-Bis-(3-methoxyphenyl)-3-methylaza-7-cyan-nonadecan-hydrogenoxalat, Fp = 97 bis 98 °C. Das Hydrochlorid-monohydrat schmilzt bei 60 bis 60,5 °C.

4. 1,7-Bis-(4-methoxyphenyl)-3-methylaza-7-cyan-nonadecan-hydrochlorid, Fp = 114 bis 116 °C.

5. 1,7-Diphenyl-3-methylaza-7-cyan-nonadecan-hydrochlorid, Fp = 112 bis 115 °C.

6. 1,7-Bis-(3-methoxyphenyl)-3-methylaza-7-cyan-docosanhydrogenoxalat, Fp = 100 bis 102 °C.

7. 1-Phenyl-3-methylaza-7-cyan-7-(3-methoxyphenyl)-nonadecan-hydrogenoxalat, Fp = 91 bis 93 °C.

8. 1-(3-Methoxyphenyl)-3-methylaza-7-cyan-7-phenyl-nonadecan-hydrogenoxalat, Fp = 100 bis 102 °C.

9. 1,7-Bis-(3-ethoxyphenyl)-3-methylaza-7-cyan-docosanhydrochlorid, Fp = 110 bis 113 °C.

10. 1,7-Diphenyl-3-methylaza-7-cyan-hexadecan-hydrochlorid, Fp = 109 bis 111 °C.

11. 1-(3-Methoxyphenyl)-3-methylaza-7-cyan-7-phenyl-hexadecan.
Analyse :

| | | | |
|---|---|---|---|
| ber. : | C 71,3 | H 8,6 | N 5,2 |
| gef. : | 71,1 | 8,6 | 5,2 |

12. 1-Phenyl-3-methylaza-7-cyan-7-(3-methoxyphenyl)-hexadecan
Analyse :

| | | | |
|---|---|---|---|
| ber. : | C 71,3 | H 8,6 | N 5,2 |
| gef. : | 71,0 | 8,6 | 5,3 |

13. 1,7-Bis-(3-methoxyphenyl)-3-methylaza-7-cyan-hexadecan
Analyse :

| | | | |
|---|---|---|---|
| ber. : | C 69,7 | H 8,5 | N 4,9 |
| gef. : | 69,7 | 8,5 | 5,0 |

14. 1-(3-Chlorphenyl)-3-methylaza-7-cyan-7-(3-methoxyphenyl)-nonadecan-hydrochlorid-monohydrat, Fp = 68 bis 71 °C.

15. 1-(3-Methoxyphenyl)-3-methylaza-7-cyan-7-(4-chlorphenyl)-nonadecan
Analyse :

| | | | | |
|---|---|---|---|---|
| ber. : | C 75,5 | H 9,4 | N 5,3 | Cl 6,8 |
| gef. : | 75,5 | 9,4 | 5,3 | 6,6 |

16. 1-(3-Methoxyphenyl)-3-methylaza-7-cyan-7-(1,3-benzodioxanyl-6)-nonadecan
Analyse :

| | | | |
|---|---|---|---|
| ber. : | C 76,6 | H 9,6 | N 5,1 |
| gef. : | 76,6 | 9,6 | 5,2 |

17. 1-Phenyl-3-methylaza-7-cyan-7-(1,3-benzodioxanyl-6)-nonadecan
Analyse :

| | | | |
|---|---|---|---|
| ber. : | C 78,7 | H 9,7 | N 5,4 |
| gef. : | 78,4 | 9,7 | 5,5 |

18. 1-(3-Methoxyphenyl)-3-methylaza-7-cyan-7-(3-trifluormethyl-phenyl)-nonadecan
Analyse :

| | | | |
|---|---|---|---|
| ber. : | C 73,0 | H 8,8 | N 5,0 |
| gef. : | 73,1 | 8,8 | 5,0 |

19. 1-(3-Methoxyphenyl)-3-methylaza-7-cyan-7-(3,4-dimethoxyphenyl)-nonadecan-hydrochlorid Fp = 96 bis 98 °C.

20. 1-(3-Methoxyphenyl)-3-methylaza-7-cyan-7-(3,4,5-trimethoxyphenyl)-nonadecan-hydrochlorid, Fp = 93 bis 95 °C.

21. 1,7-Bis-(3-chlorphenyl)-3-methylaza-7-cyan-nonadecan
Analyse :

| | | | | |
|---|---|---|---|---|
| ber. : | C 72,6 | H 8,8 | N 5,3 | Cl 13,4 |
| gef. : | 72,8 | 8,6 | 5,5 | 13,4 |

22. 1-(3-Methoxyphenyl)-3-methylaza-7-cyan-7-(3-chlorphenyl)-nonadecan
Analyse :

| | | | | |
|---|---|---|---|---|
| ber. : | C 75,5 | H 9,4 | N 5,3 | Cl 6,8 |
| gef. : | 75,6 | 9,4 | 5,3 | 6,8 |

23. 1-(3-Methoxyphenyl)-3-methylaza-7-cyan-7-(3,4-ethylendioxyphenyl)-nonadecan
Analyse :

| | | | |
|---|---|---|---|
| ber. : | C 76,6 | H 9,6 | N 5,1 |
| gef. : | 76,4 | 9,3 | 5,1 |

24. 1-(3-Methoxyphenyl)-3-methylaza-7-cyan-7-(3-tolyl)-nonadecan
Analyse :

| | | | |
|---|---|---|---|
| ber. : | C 80,9 | H 10,4 | N 5,5 |
| gef. : | 80,6 | 10,1 | 5,5 |

25. 1,7-Bis-(3-Methoxyphenyl)-3-methylaza-7-cyan-octadecan
Analyse :

| | | | |
|---|---|---|---|
| ber. : | C 78,2 | H 9,9 | N 5,5 |
| gef. : | 78,1 | 9,8 | 5,5 |

26. 1.7-Bis-(3-Methoxyphenyl)-3-methylaza-7-cyan-8-(n-butyl)-dodecan
Analyse :

| | | | |
|---|---|---|---|
| ber. : | C 77,8 | H 9,7 | N 5,9 |
| gef. : | 77,8 | 9,7 | 5,9 |

27. 1,7-Bis-(3-Methoxyphenyl)-3-methylaza-7-cyan-8-(n-pentyl)-tridecan
Analyse :

| | | | |
|---|---|---|---|
| ber. : | C 78,2 | H 9,9 | N 5,5 |
| gef. : | 78,2 | 10,0 | 5,4 |

28. 1,7-Bis-(3-Methoxyphenyl)-3-methylaza-7-cyan-8-(n-hexyl)-tetradecan
Analyse :

| | | | |
|---|---|---|---|
| ber. : | C 78,6 | H 10,2 | N 5,2 |
| gef. : | 78,7 | 9,8 | 5,2 |

29. 1-(3-Methoxyphenyl)-3-methylaza-7-cyan-7-(3-n-butoxyphenyl)-nonadecan
Analyse :

| | | | |
|---|---|---|---|
| ber. : | C 78,9 | H 10,4 | N 5,0 |
| gef. : | 78,8 | 10,5 | 5,2 |

30. 1-(3-n-Butoxyphenyl)-3-methylaza-7-cyan-7-(3-methoxyphenyl)-nonadecan
Analyse :

| | | | |
|---|---|---|---|
| ber. : | C 78,9 | H 10,4 | N 5,0 |
| gef. : | 78,8 | 10,3 | 5,1 |

31. 1,7-Bis-(3-n-butoxyphenyl)-3-methylaza-7-cyan-nonadecanhydrochlorid Fp = 127-129 °C

32. 1.7-Bis-(3-Methoxyphenyl)-3-methylaza-7-cyan-17-octadecan
Analyse :

| | | | |
|---|---|---|---|
| ber. : | C 78,5 | H 9,6 | N 5,5 |
| gef. : | 78,7 | 9,6 | 5,4 |

33. 1-(3-Methoxyphenyl)-3-methylaza-7-cyan-(3-fluorphenyl)-nonadecan
Analyse :

| | | | |
|---|---|---|---|
| ber. : | C 77,9 | H 9,7 | N 5,5 |
| gef. : | 78,0 | 9,7 | 5,5 |

34. 1-(3-Fluorphenyl)-3-methylaza-7-cyan-7-(3-methoxyphenyl)-nonadecan
Analyse :

| | | | |
|---|---|---|---|
| ber. : | C 77,9 | H 9,7 | N 5,5 |
| gef. : | 78,0 | 9,6 | 5,4 |

35. 1.7-Bis-(3-Fluorphenyl)-3-methylaza-7-cyan-nonadecan
Analyse :

| | | | |
|---|---|---|---|
| ber. : | C 77,4 | H 9,3 | N 5,6 |
| gef. : | 77,2 | 9,3 | 5,6 |

36. 1-(3-Methoxyphenyl)-3-methylaza-7-cyan-7-(3-tert.-butoxyphenyl)-nonadecan
Analyse :

| | | | |
|---|---|---|---|
| ber. : | C 79,0 | H 10,4 | N 5,0 |
| gef. : | 78,8 | 10,3 | 4,9 |

37. 1-(3-tert.-Butoxyphenyl)-3-methylaza-7-cyan-7-(3-methoxyphenyl)-nonadecan
Analyse :

| | | | |
|---|---|---|---|
| ber. : | C 79,0 | H 10,4 | N 5,0 |
| gef. : | 78,9 | 10,5 | 5,0 |

38. 1.7-Bis-(3-tert.-Butoxyphenyl)-3-methylaza-7-cyan-nonadecan
Analyse :

| | | | |
|---|---|---|---|
| ber. : | C 79,4 | H 10,7 | N 4,6 |
| gef. : | 79,5 | 10,6 | 4,7 |

In analoger Weise können erhalten werden :

39. 1-(4-tert-Butylphenyl)-3-methylaza-7-cyan-7-(3-methoxyphenyl)-nonadecan

40. 1-(4-Fluorphenyl)-3-methylaza-7-cyan-7-(3-methoxyphenyl)-nonadecan

41. 1-(3,4-Dimethoxyphenyl)-3-methylaza-7-cyan-7-(4-bromphenyl)-eicosan

42. 1-(4-Ethylmercaptophenyl)-3-methylaza-7-cyan-7-(3-methoxyphenyl)-nonadecan

43. 1,7-Bis-(3-methylmercaptophenyl)-3-methylaza-7-cyan-nonadecan

44. 1-(3,4-Dichlorphenyl)-3-methylaza-7-cyan-7-(3-methoxyphenyl)-nonadecan

45. 1-(3-Fluorphenyl)-3-methylaza-7-cyan-7-(3-chlorphenyl)-nonadecan

## Beispiel 46

1,7-Bis-(3,4-dimethoxyphenyl)-3-methylaza-7-cyan-nonadecan

In einem Dreihalskolben, der mit Rührer, Tropftrichter und Rückflußkühler versehen war, wurden 34,5 g (0,1 mol) alpha-Dodecylveratrylcyanid in 15 ml Toluol bei 40 °C gelöst. Zu dieser Lösung gab man 26 g Kaliumhydroxidpulver und 0,2 g Tetrabutylammoniumiodid. Anschließend wurde unter Rühren eine Lösung von 27 g (0,1 Mol) N-Methyl-N-homoveratryl-amino-gamma-chlorpropan in 20 ml Toluol in dem Maße zugegeben, daß die Reaktionstemperatur 90 °C nicht überstieg. Nach beendeter Zugabe wurde weitere 2,5 h bei dieser Temperatur nachgerührt. Das erkaltete Reaktionsgemisch wurde mit 100 ml Wasser gewaschen. Nach dem Abdestillieren des Lösungsmittels erhielt man 55 g Endprodukt als gelbes Öl. Dessen Hydrogenoxalat schmilzt bei 93 bis 96 °C.
In analoger Weise wurden erhalten :

47. 1-(3,4-Dimethoxyphenyl)-3-methylaza-7-cyan-7(3-chlorphenyl)-nonadecan
Analyse :

| | | | | |
|---|---|---|---|---|
| ber. : | C 73,5 | H 9,3 | N 5,0 | Cl 6,4 |
| gef. : | 73,6 | 9,3 | 5,0 | 6,4 |

48. 1-(3,4-Dilmethoxyphenyl)-3-methylaza-7-cyan-7-(1,3-benzodioxanyl-6)-nonadecan
Analyse :

| | C | H | N |
|---|---|---|---|
| ber. : | 74,7 | 9,4 | 4,8 |
| gef. : | 74,6 | 9,2 | 4,9 |

49. 1-(3,4-Dimethoxyphenyl)-3-methylaza-7-cyan-7-(3,4,5-trimethoxyphenyl)-nonadecan-amidosulfonat Fp = 99 bis 102 °C.

50. 1-(3,4-Dimethoxyphenyl)-3-methylaza-7-cyan-7-(3-ethoxyphenyl)-docosan-hydrochlorid, Fp = 109 bis 112 °C.

51. 1-(3,4-Dimethoxyphenyl)-3-methylaza-7-cyan-7-(3-ethoxyphenyl)-nonadecan-hydrochlorid, Fp = 111 bis 113 °C.

52. 1-(3,4-Dimethoxyphenyl)-3-methylaza-7-cyan-7-(3,4,5-trimethoxy-phenyl)-pentacosan-hydrochlorid, Fp = 98 bis 101 °C.

53. 1-(3,4-Dimethoxyphenyl)-3-methylaza-7-cyan-(3,4,5-trimethoxyphenyl)-heptadecan-amidosulfonat Fp = 97 bis 100 °C.

## Beispiel 54

1,6-Bis-(3,4-Dimethoxyphenyl)-3-ethylaza-6-cyan-oktadecan

45,2 g (0,1 Mol) alpha-(n-Dodecyl)-alpha-(2-bromethyl)-3,4-dimethoxyphenyl-acetonitril (erhalten durch Kondensation von alpha-Dodecyl-3,4-dimethoxy-phenylacetonitril mit 1,2-Dibromethan in toluolischer Lösung in Gegenwart von Lithiumdiisopropylamid) und 41,8 g beta-(N-Ethyl)-3,4-dimethoxyphenethylamin wurden zwei h im Ölbad auf 150 °C erhitzt. Man versetzte noch heiß mit 250 ml Toluol und saugte den sich abscheidenden Niederschlag von beta-(N-Ethyl)-3,4-dimethoxy-phenethylamin-hydrobromid ab.

Das Filtrat wurde mit 2N-Natronlauge gewaschen, dann die Base mit methanolischer wäßriger amidosulfonsäure ausgeschüttelt und diese mehrfach mit Ether gewaschen. Die Base wurde durch Zugabe von Kaliumcarbonatlösung freigesetzt, in Ether aufgenommen und die Lösung mit wasserfreiem Kaliumcarbonat getrocknet. Nach dem Abdestillieren des Lösungsmittels hinterblieben 42 g (72 %) zähflüssiges gelbes Öl, das säulenchromatographisch (Kieselgel, Essigsäureethylester) gereinigt wurde.
Analyse :

| | C | H | N |
|---|---|---|---|
| ber. : | 74,4 | 9,7 | 4,8 |
| gef. : | 74,3 | 9,6 | 4,8 |

In analoger Weise erhielt man :

55. 1,7-Bis-(3,5-di-n-butoxyphenyl)-3-methylaza-7-cyan-nonadecan
Analyse :

| | C | H | N |
|---|---|---|---|
| ber. : | 76,9 | 10,8 | 3,7 |
| gef. : | 76,9 | 10,9 | 4,0 |

56. 1,7-Bis-(3,5-di-isopropoxyphenyl)-3-methylaza-7-cyan-nonadecan
Analyse :

| | C | H | N |
|---|---|---|---|
| ber. : | 76,2 | 10,5 | 4,0 |
| gef. : | 76,3 | 10,4 | 4,0 |

57. 1,7-Bis-(3,5-di-n-propoxyphenyl)-3-methylaza-7-cyan-nonadecan
Analyse :

| | C | H | N |
|---|---|---|---|
| ber. : | 76,3 | 10,5 | 4,0 |
| gef. : | 76,4 | 10,4 | 4,0 |

58. 1-(3-Chlorphenyl)-3-methylaza-7-cyan-7-(1,3-benzodioxanyl-6)-nonadecan
Analyse :

| | C | H | N | Cl |
|---|---|---|---|---|
| ber. : | 73,8 | 8,9 | 5,1 | 6,4 |
| gef. : | 73,7 | 8,8 | 5,2 | 6,4 |

59. 1-(3-Trifluormethylphenyl)-3-methylaza-7-cyan-7-(3-methoxyphenyl)-nonadecan
Analyse :

| | C | H | N |
|---|---|---|---|
| ber. : | 73,0 | 8,8 | 5,0 |
| gef. : | 73,2 | 8,8 | 5,0 |

60. 1-(3-Chlorphenyl)-3-methylaza-7-cyan-7-(3,4-dimethoxyphenyl)-nonadecan
Analyse :

| | | | | |
|---|---|---|---|---|
| ber. : | C 73,5 | H 9,3 | N 5,0 | Cl 6,4 |
| gef. : | 73,7 | 9,2 | 4,9 | 6,5 |

61. 1-(3,5-Dimethoxyphenyl)-3-methylaza-7-cyan-7-(3-trifluormethylphenyl)-nonadecan
Analyse :

| | | | |
|---|---|---|---|
| ber. : | C 71,4 | H 8,7 | N 4,8 |
| gef. : | 71,6 | 9,0 | 4,8 |

62. 1-(3,4-Methylendioxyphenyl)-3-methylaza-7-cyan-7-(3,4-dimethoxyphenyl)-nonadecan
Analyse :

| | | | |
|---|---|---|---|
| ber. : | C 74,4 | H 9,3 | N 5,0 |
| gef. : | 74,6 | 9,2 | 5,1 |

63. 1-(3,4-Ethylendioxyphenyl)-3-methylaza-7-cyan-7-(3-methoxyphenyl)-nonadecan
Analyse :

| | | | |
|---|---|---|---|
| ber. : | C 76,6 | H 9,6 | N 5,1 |
| gef. : | 76,7 | 9,6 | 5,1 |

64. 1-(1,3-Benzodioxanyl-6)-3-methylaza-7-cyan-7-(3,5-diethoxyphenyl)-nonadecan
Analyse :

| | | | |
|---|---|---|---|
| ber. : | C 75,2 | H 9,6 | N 4,6 |
| gef. : | 75,1 | 9,5 | 4,8 |

65. 1-(3-Nitrophenyl)-3-methylaza-7-cyan-7-(3-methoxyphenyl)-nonadecan
Analyse :

| | | | |
|---|---|---|---|
| ber. : | C 74,0 | H 9,2 | N 7,8 |
| gef. : | 74,1 | 9,1 | 7,9 |

66. 1,7-Bis-(3,5-diethoxyphenyl)-3-methylaza-7-cyan-nonadecan
Analyse :

| | | | |
|---|---|---|---|
| ber. : | C 75,1 | H 10,1 | N 4,4 |
| gef. : | 75,5 | 10,0 | 4,4 |

Beispiel 67 bis 73

Reduktive Aminierung von alpha-Alkyl-alpha-(gamma-oxopropyl)-phenylacetonitrilen mit N-Methyl-betaphenethylaminen.

0,5 Mol des entsprechenden alpha-Alkyl-alpha-(gamma-oxopropyl)-phenylacetonitrils (hergestellt aus den alpha-Alkyl-phenylacetonitrilen und beta-Chlorpropionaldehyddiethylacetal in Gegenwart von Lithium-diisopropylamid und anschließende Freisetzung des Aldehyds mit wäßriger Oxalsäurelösung) und 0,5 Mol N-Alkyl-beta-phenethylamin werden in 500 ml Toluol gelöst. Dazu gibt man in der Kälte 0,55 Mol Ameinsäure (98 bis 100 %ig). Das Reaktionsgemisch wird bis zum Abklingen der Gasentwicklung unter Rückfluß erhitzt.

Die erkaltete Reaktionslösung wird mit wäßriger Kaliumcarbonatlösung versetzt, das freigesetzte Amin mit einem Ether/Hexan-Gemisch extrahiert und daraus anschließend mit wäßriger Amidosulfonsäure ausgezogen. Zur Entfernen von Neutralbestandteilen wird die Amidosulfonatlösung mehrmals mit Ether extrahiert, die Base mit Kaliumcarbonatlösung freigesetzt und mit Ether extrahiert. Die etherische Lösung wird nach dem Trocknen mit Kaliumcarbonat im Vakuum abdestilliert und der ölige Rückstand über Salzbildung und Kristallisation bzw. durch Säulenchromatographie (Kieselgel, Essigsäureethylester) gereinigt. Die Ausbeuten liegen um 85 %.

Nach diesem Verfahren wurden die folgenden Verbindungen hergestellt :

67. 1-(4-Chlorphenyl)-3-methylaza-7-cyan-7-(3-methoxyphenyl)-nonadecan-hydrochlorid, Fp = 89 bis 90 °C.

68. 1-(3,5-Diethoxyphenyl)-3-methylaza-7-cyan-7-(3-ethoxyphenyl)-docosan-hydrochlorid, Fp = 80 bis 83 °C.

69. 1-(3,5-Dimethoxyphenyl)-3-methylaza-7-cyan-7-(3-ethoxyphenyl)-docosan-hydrochlorid, Fp = 83 bis 86 °C.

70. 1-(3,5-Dimethoxyphenyl)-3-methylaza-7-cyan-7-(3-ethoxyphenyl)-nonadecan-hydrochlorid, Fp = 82 bis 85 °C.

71. 1-(3,4,5-Trimethoxyphenyl)-3-methylaza-7-cyan-7-(3-ethoxyphenyl)-docosan-hydrochlorid, Fp = 119 bis 120 °C.

72. 1-(3,4,5-Trimethoxyphenyl)-3-methylaza-7-cyan-7-(3-ethoxyphenyl)-nonadecan-hydrochlorid, Fp = 116 bis 118 °C.

73. 1-(3,5-Diethoxyphenyl)-3-methylaza-7-cyan-7-(3-ethoxyphenyl)-nonadecan-hydrochlorid, Fp = 77 bis 79 °C.

Beispiel 74

1,7-Bis-(3-methoxyphenyl)-3-aza-7-cyan-eicosan

37 g alpha-Tridecyl-alpha-(gamma-oxopropyl)-3-methoxyphenyl-acetonitril und 15 g beta-(3-Methoxyphenyl)-ethylamin wurden in 500 ml Toluol gelöst und so lange unter Rückfluß gekocht, bis am Wasserabscheider keine Abscheidung von Wasser mehr feststellbar war. Anschließend wurde das Toluol abdestilliert, der ölige Rückstand in 500 ml Methanol gelöst und unter Rühren bei Raumtemperatur mit 3,8 g Natriumborhydrid versetzt. Nach weiteren 5 h Rühren wurde Wasser zugegeben, mit einem Etherhexan-Gemisch extrahiert und mit wäßriger Amidosulfonsäurelösung geschüttelt. Die abgetrennte Amidosulfonsäurephase wurde mit wäßriger Kaliumcarbonatlösung alkalisiert und die ausgeschiedene Base mit Ether extrahiert. Nach Trocknen mit Kaliumcarbonat und Abdestillieren des Ethers wurde der ölige Rückstand säulenchromatographisch (Kieselgel, Essigsäure-ethylester) gereinigt.

Analyse :

| | | | |
|---|---|---|---|
| ber. : | C 78,4 | H 10,1 | N 5,4 |
| gef. : | 78,5 | 10,0 | 5,2 |

In analoger Weise wurde erhalten :

75. 1,7-Bis-(3-methoxyphenyl)-3-aza-7-cyan-nonadecan-hydrogenoxalat Fp = 121 bis 123 °C.

Beispiel 76

1,8-Diphenyl-3-methylaza-8-cyan-eicosan

Ein Gemisch aus 0,5 Mol Alpha-Dodecyl-alpha-(delta-methylaminobutyl)-phenyl-acetonitril (hergestellt durch Umsetzung von alpha-Dodecyl-phenylacetonitril und 4-Methylformamido-1-chlorbutan in Gegenwart von Natriumhydrid und anschließende Abspaltung der Formylgruppe mittels Salzsäure), 0,5 Mol Phenylacetaldehyd, 2 g 5 %igem Palladium auf Kohle und 500 ml Toluol wurde katalytisch unter Atmosphärendruck bei 25 bis 30 °C während 20 h reduziert. Nach Entfernung des Katalysators wurde die Base mit wäßriger Amidosulfonsäurelösung extrahiert, die Säurelösung mit Ether gewaschen und anschließend mit wäßriger Kaliumcarbonatlösung die Base wieder freigesetzt. Diese wurde mit Ether extrahiert, der Extrakt mit Kaliumcarbonat getrocknet und anschließend der Ether abdestilliert. Die erhaltene Base wurde durch Säulenchromatographie (Kieselgel, Essigsäureethylester) gereinigt.

Analyse :

| | | | |
|---|---|---|---|
| ber. : | C 83,5 | H 10,6 | N 5,9 |
| gef. : | 83,4 | 10,3 | 5,7 |

Analog erhält man :

77. 1,8-Diphenyl-4-methylaza-8-cyan-eicosan.

Analyse :

| | | | |
|---|---|---|---|
| ber. : | C 83,5 | H 10,6 | N 5,9 |
| gef. : | 83,7 | 10,4 | 5,7 |

Beispiel 78

(+)-1,7-Bis-(3-methoxyphenyl)-3-methylaza-7-cyan-nonadecan

38,7 g (0,1 Mol) 4-Cyan-4-(3-methoxyphenyl)-hexadecancarbonsäure (Fp. 36-38 °C, hergestellt durch Anlagerung von Acrylnitril an alpha-Dodecyl-3-methoxybenzylcyanid und anschließende Verseifung) wurden mit 29,4 g (0,1 Mol) Cinchonin in 200 ml Methanol gelöst. Nach einiger Zeit kristallisierte das Salz der rechtsdrehenden Säure aus, welches abgesaugt und mehrmals bis zur Drehwertkonstanz aus 3 Teilen Methanol umkristallisiert wurde. Ausbeute nach zweimaligem Umkristallisieren 12 g (35 %), Fp = 125-127 °C, $[\alpha]_{589}^{20}$ nm = + 98° (Ethanol, C = 10 mg/ml).

Aus dem Salz erhält man in üblicher Weise die freie rechtsdrehende Säure, Fp. 41 °C, $[\alpha]_{589}^{20}$ nm = + 15° (Ethanol, C = 15 mg/ml).

Die Säure wurde durch Umsetzung mit Chlorameisensäureethylester in das gemischte Anhydrid überführt, welches in guter Ausbeute mit Natriumborhydrid zu (+)-4-(3-Methoxyphenyl)-4-cyan-hexadecanol reduziert wurde. Diese wurde roh mit Thionylchlorid zu (+)-4-(3-Methoxyphenyl)-4-cyan-1-chlorhexadecan umgesetzt. Beide Umsetzungen erfolgten in analoger Weise wie in den DE-PS 20 59 923 und DE-PS 20 59 985 beschrieben.

5 g (0,012 8 Mol) rohes (+)-4-(3-Methoxyphenyl)-4-cyan-1-chlorhexadecan wurden sodann mit 4,2 g (0,025 4 Mol) N-Methyl-3-methoxyphenethylamin 1 h auf 160 °C erhitzt, die erkaltete Reaktionsmischung mit Ether versetzt, vom ausgeschiedenen Hydrochlorid abgesaugt, die Lösung mit Wasser gewaschen, getrocknet und eingedampft. Der Rückstand wurde säulenchromatographisch (Kieselgel, Essigsäureethylester) gereinigt. Ausbeute : 4,8 g (69,5 %) gelbliches Öl, Rf = 0,8 ($CH_3Cl$/Methanol 9 : 1), 0,2 (Hexan/Essigsäureethylester 6 : 4), $[\alpha]_{589}^{20}$ nm = + 7° (Benzol, C = 30 mg/ml).

## Beispiel 79

(−)-1,7-Bis-(3-methoxyphenyl)-3-methylaza-7-cyan-nonadecan

Aus den Mutterlaugen der 1. Stufe des Beispiels 78 wurde die linksdrehende Säure (−)-4-Cyan-4-(3-methoxyphenyl)hexadecan-carbonsäure in geringer optischer Reinheit erhalten. Diese wurde mit Brucin in 90 %igem wäßrigem Methanol zur Kristallisation angesetzt und das Brucinsalz der linksdrehenden Saüre erhalten, das ebenfalls bis zur Drehwertkonstanz umkristallisiert wurde. Fp. 61 °C, $[\alpha]_{589}^{20}$ nm = 15,5° (Ethanol, C = 10 mg/ml).

Daraus wurde die linksdrehende Säure rein isoliert :

Fp. 41 °C, $[\alpha]_{589}^{20}$ nm = 16° (Ethanol, C = 10 mg/ml).

Die weiteren Syntheseschritte wurden analog Beispiel 78 durchgeführt. Die Eigenschaften der gewünschten Verbindung stimmten auf den spezifischen Drehwert $[\alpha]_{589}^{20}$ nm = − 7° (Benzol C = 30 mg/ml) mit dem in Beispiel 78 beschriebenen Enantiomeren überein.

## Beispiel 80

1,7-Bis-(3-hydroxyphenyl)-3-methylaza-7-cyan-nonadecan

60,5 g (0,1 Mol) 1,7-Bis-(3-tert.-butoxyphenyl)-3-methylaza-7-cyan-nonadecan (Beispiel 38) wurden mit 200 ml wasserfreier Trifluoressigsäure übergossen und 4 h bei Raumtemperatur stehengelassen. Anschließend wurde wie Trifluoressigsäure im Vakuum abdestilliert, der Rückstand mit 500 ml Wasser versetzt und mit Ammoniak die Base freigesetzt. Die Base wurde mit 200 ml Ether aufgenommen, mit Wasser gewaschen und mit Magnesiumsulfat getrocknet. Nach Abdestillieren des Ethers wurde die als öliger Rückstand erhaltene Base an Kieselgel mit Essigester/Methanol (95/5) chromatographiert.

Analyse :

| | | | |
|---|---|---|---|
| ber. : | C 78,0 | H 9,8 | N 5,7 |
| gef. : | 77,8 | 9,7 | 5,6 |

Analog wurden erhalten :

81. 1-(3-Methoxyphenyl)-3-methylaza-7-cyan-7-(3-hydroxyphenyl)-nonadecan

Analyse :

| | | | |
|---|---|---|---|
| ber. : | C 78,2 | H 9,9 | N 5,5 |
| gef. : | 78,3 | 9,8 | 5,4 |

82. 1-(3-Hydroxyphenyl)-3-methylaza-7-cyan-7-(3-methoxyphenyl)-nonadecan

Analyse :

| | | | |
|---|---|---|---|
| ber. : | C 78,2 | H 9,9 | 5,5 |
| gef. : | 78,1 | 9,7 | 5,5 |

## Beispiel 83

1-(3-Nitro-4-methoxyphenyl)-3-methylaza-7-cyan-7-(3-methoxyphenyl)-nonadecan.

20,0 g (0,054 Mol) N-Methyl-4-cyano-4-(3-methoxyphenyl)-hexadecylamin (Hydrochlorid : Fp = 112 bis 114 °C), 5,8 g (0,027 Mol) 3-Nitro-4-methoxyphenethylchlorid und 0,25 g Natriumiodid wurden in einem Lösungsmittelgemisch aus 50 ml Acetonitril und 10 ml Dimethylsulfoxid 60 h auf 50 bis 55 °C erwärmt. Nach Zugabe von 100 ml Wasser wurde die Reaktionslösung mit Ammoniak alkalisch gemacht und anschließend die Base mit einem Gemisch aus 350 ml n-Hexan und 50 ml Ether extrahiert. Nach

Abdestillieren der Lösungsmittel wird die als öliger Rückstand erhaltene Base an Kieselgel mit Essigester chromatographiert.

Analyse :

| | | | |
|---|---|---|---|
| ber. : | C 72,2 | H 9,1 | 7,4 |
| gef. : | 72,1 | 9,0 | 7,5 |

Analog wurde erhalten :

84. 1-(2-Chlorphenyl)-3-methylaza-7-cyan-7-(3-methoxyphenyl)-nonadecan

Analyse :

| | | | | |
|---|---|---|---|---|
| ber. : | C 75,5 | H 9,4 | N 5,3 | Cl 6,8 |
| gef. : | 75,5 | 9,3 | 5,5 | 6,9 |

## Beispiel 85

1-(3-Methoxyphenyl)-3-methylaza-7-cyan-7-(3,4,5-trimethoxyphenyl)-eicosan

20,7 g (0,1 Mol) 3,4,5-Trimethoxyphenylacetonitril werden bei 40 °C in 15 ml Toluol gelöst und mit 52 g (0,8 Mol) 85 %igem Kaliumhydroxidpulver und 0,2 g Tetrabutylammoniumiodid versetzt. Anschließend wurde unter Rühren eine Lösung von 26,3 g (0,1 Mol) Tridecylbromid in 20 ml Toluol so zugegeben, daß die Reaktionstemperatur 90 °C nicht überstieg. Nach beendeter Zugabe wurde 2 h bei dieser Temperatur nachgerührt und anschließend eine Lösung von 24,2 g (0,1 Mol) 1-Chlor-4-methylaza-6-(3-methoxyphenyl)-hexan in 20 ml Toluol bei 90 °C zugegeben. Die Reaktionsmischung wurde weitere 3 h bei 90 °C gerührt, nach Erkalten mit 100 ml Wasser versetzt und die Toluolphase abgetrennt. Nach Abdestillieren des Lösungsmittels erhielt man die Rohbase als gelbes Öl, das an Kieselgel mit Essigester chromatographiert wird.

Rf-Wert (Methylenchlorid/Methanol 93/7) : 0,44.

Analyse :

| | | | |
|---|---|---|---|
| ber. : | C 74,7 | H 9,8 | N 4,7 |
| gef. : | 74,7 | 9,4 | 4,8 |

## Beispiel 86

1-(3-Methoxyphenyl)-3-methylaza-7-cyan-7-(3,4-dimethoxy-phenyl)-eicosan

38,3 g (0,1 Mol) 1-(3-Methoxyphenyl)-3-methylaza-7-cyan-7-(3,4-dimethoxyphenyl)-heptan wurden in 200 ml Toluol gelöst und mit 4,7 g (0,12 Mol) pulverisiertem Natriumamid 1 h unter Rühren und Rückfluß gekocht. Anschließend tropfte man innerhalb von 60 min die Lösung von 26,3 g (0,1 Mol) Tridecylbromid in 50 ml Toluol hinzu und erhitzte noch 2 h unter Rückfluß. Die erkaltete Reaktionsmischung wurde in Wasser gegossen, die Toluolphase mehrmals mit Wasser gewaschen und anschließend das Toluol abdestilliert. Der erhaltene Rückstand wurde an Kieselgel mit Essigester chromatographiert.

Rf-Wert (Methylenchlorid/Methanol = 93/7) : 0,44

Analyse :

| | | | |
|---|---|---|---|
| ber. : | C 76,6 | H 10,0 | N 5,0 |
| gef. : | 76,4 | 9,3 | 5,0 |

## Beispiel 87

1,7-Bis-(3-methoxyphenyl)-3-methylaza-7-cyan-eicosan

52,1 g (0,1 Mol) der nach Beispiel 74 erhaltenen Verbindung wurden in der Kälte in 23 g (0,5 Mol) Ameisensäure gelöst und nach Zugabe von 11,9 ml 35 %iger wäßriger Formalinlösung (0,15 Mol Formaldehyd) bis zur Beendigung der Kohlendioxidentwicklung auf dem Wasserbad erhitzt. Nach dem Abkühlen wurde die Reaktionslösung mit Wasser verdünnt, durch Zugabe von Ammoniak alkalisch gemacht und mit n-Hexan die abgeschiedene Base extrahiert. Die n-Hexanlösung wurde mehrmals mit Wasser gewaschen, mit Kaliumcarbonat getrocknet und anschließend das n-Hexan abdestilliert.

Man chromatographierte den Rückstand an Kieselgel (Essigester/ n-Hexan = 7/3) und erhielt nach Einengung der Hauptfraktion die Base als zähviskoses Öl.

Rf-Wert (Methylenchlorid/Methanol = 93/7) : 0,53.

Analyse :

| | | | |
|---|---|---|---|
| ber. : | C 78,6 | H 10,2 | N 5,2 |
| gef. : | 78,6 | 10,1 | 5,1 |

## Beispiel 88

Auf einer Tablettenpresse werden in üblicher Weise Tabletten folgender Zusammensetzung gepreßt :

40 mg Substanz des Beispiels 3
120 mg Maisstärke
13,5 mg Gelatine
45 mg Milchzucker
2,25 mg Aerosil® (chemisch eine Kieselsäure in submikroskopisch feiner Verteilung)
6,75 mg Kartoffelstärke (als 6 %iger Kleister).

Beispiel 89

In üblicher Weise werden Dragees folgender Zusammensetzung hergestellt :

20 mg Substanz des Beispiels 3
60 mg Kernmasse
60 mg Verzuckerungsmasse

Die Kernmasse besteht aus 9 Teilen Maisstärke, 3 Teilen Milchzucker und 1 Teil Luviskol® VA 64 (Vinylpyrrolidon-Vinylacetat-Mischpolymerisat 60 : 40, vgl. Pharm. Ind. *1962*, 586). Die Verzuckerungsmasse besteht aus 5 Teilen Rohrucker, 2 Teilen Maisstärke, 2 Teilen Calciumcarbonat und 1 Teil Talk. Die so hergestellten Dragees werden anschließend mit einem magensaftresistenten Überzug versehen.

Beispiel 90

10 g Substanz des Beispiels 3 werden in 5 000 ml Wasser unter Zusatz von NaCl gelöst und mit 0,1 N NaOH auf pH 6,0 eingestellt, so daß eine blutisotonische Lösung entsteht. Jeweils 5 ml dieser Lösung werden in Ampullen gefüllt und sterilisiert.

**Ansprüche**

1. omega-Cyan-1,.omega-diphenyl-azaalkan-derivate der allgemeinen Formel I,

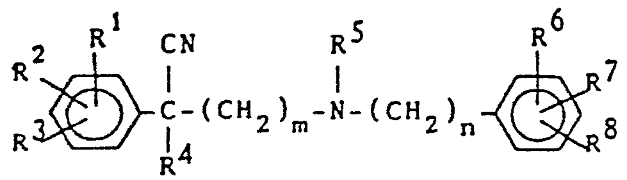

(I)

worin
$R^1$, $R^2$, $R^3$, $R^6$, $R^7$, $R^8$ gleich oder verschieden sind und Wasserstoffatome, Halogenatome, Hydroxylgruppen, Trifluormethylgruppen, $C_1$-$C_4$-Alkylgruppen, Nitrogruppen, $C_1$-$C_4$-Alkoxygruppen oder $C_1$-$C_4$-Alkylmercaptogruppen bedeuten, wobei auch jeweils zwei Reste in Nachbarstellung zusammen Methylendioxy-, Ethylendioxy- oder 1,3-Dioxatetramethylengruppen bilden können,
$R^4$ eine gerade oder verzweigte, gesättigte oder ungesättigte Alkylgruppe mit 9-20 Kohlenstoffatomen ist,
$R^5$ ein Wasserstoffatom oder einen $C_1$-$C_4$-Alkylrest darstellt und
m und n gleich oder verschieden sind und die Zahlen 2 bis 4 bedeuten,
sowie deren Salze mit physiologisch verträglichen Säuren.

2. 1,7-Bis-(3-Methoxyphenyl)-3-methylaza-7-cyan-nonadecan.
3. 1-(3-Methoxyphenyl)-3-methylaza-7-cyan-7-(3,4-dimethoxyphenyl)-nonadecan.
4. 1-(3-Methoxyphenyl)-3-methylaza-7-cyan-7-(3,4,5-trimethoxyphenyl)-nonadecan.
5. 1,7-Diphenyl-3-methylaza-7-cyan-nonadecan.
6. Verfahren zur Herstellung der Verbindungen der Formel I gemäß Anspruch 1, dadurch gekennzeichnet, daß man

a) Phenylacetonitrile der Formel II,

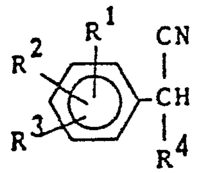

(II)

worin $R^1$ bis $R^4$ die angegebene Bedeutung haben, mit 1-Phenylazaalkanen der Formel III,

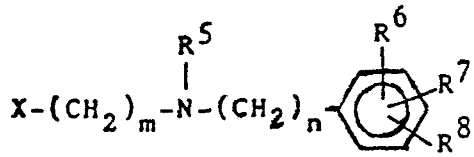

(III)

worin $R^5$ bis $R^8$ sowie m und n die angegebene Bedeutung besitzen, und X eine Austrittsgruppe darstellt, umsetzt oder

b) omega-Cyan-1, omega-diphenyl-azaalkan-derivate der Formel IV,

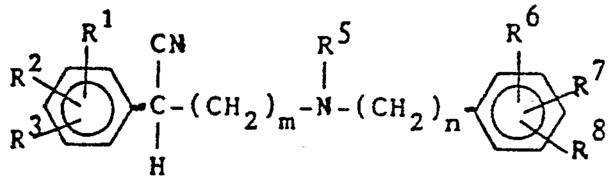

(IV)

worin $R^1$ bis $R^8$, m und n die angegebene Bedeutung haben mit Verbindungen der Formel

$$R^4—Y \quad (V)$$

worin $R^4$ die angegebene Bedeutung hat und Y eine Austrittsgruppe darstellt, zur Reaktion bringt oder

c) Phenylacetonitrile der Formel VI,

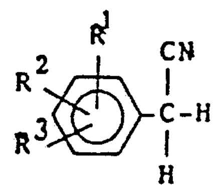

(VI)

worin $R^1$ bis $R^3$ die angegebene Bedeutung haben, mit 1-Phenylazaalkanen der Formel III und Verbindungen der Formel V umsetzt oder

d) Phenylacetonitrile der Formel VII,

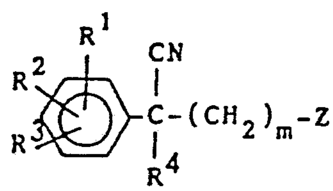

(VII)

worin $R^1$ bis $R^4$ und m die oben angegebenen Bedeutungen haben und Z eine Austrittsgruppe darstellt, mit einem Phenylalkylamin der Formel VIII,

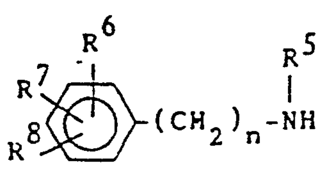

(VIII)

worin $R^5$ bis $R^8$ und n die obige Bedeutung besitzen, zur Reaktion bringt oder

e) omega-Amino-alkyl-phenylacetonitrile der Formel IX,

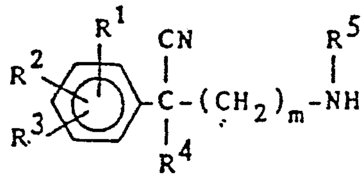

(IX)

worin $R^1$ bis $R^5$ und n die beschriebene Bedeutung haben, mit Phenylalkyl-derivaten der Formel X,

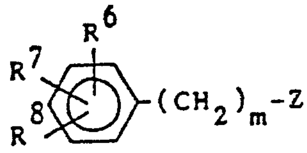

(X)

worin $R^6$ bis $R^8$, m und Z die oben angegebene Bedeutung haben, zur Reaktion bringt oder

f) omega-Cyan-1, omega-diphenyl-azaalkan-derivate der Formel XI,

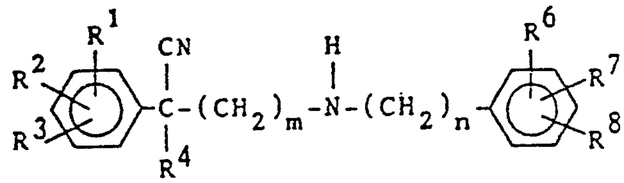

(XI)

worin $R^1$ bis $R^8$, m und n die angegebene Bedeutung haben, alkyliert oder

g) omega-Aminoalkyl-phenylacetonitrile der Formel IX mit Aldehyden der Formel XII,

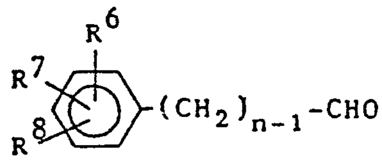

(XII)

worin $R^6$ bis $R^8$ und n dasselbe wie oben bedeutet, unter reduktiven Bedingungen umsetzt oder

h) Aldehyde der Formel XIII,

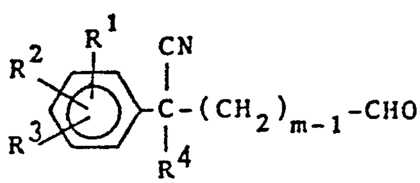

(XIII)

worin $R^1$ bis $R^4$ und m dasselbe wie oben bedeutet, mit Phenylalkylaminen der Formel VIII unter reduktiven Bedingungen umsetzt,
und die so erhaltenen Verbindungen, falls gewünscht und falls einer oder mehrere der Reste $R^1$, $R^2$, $R^3$, $R^6$, $R^7$ oder $R^8$ Alkoxygruppen sind, diese einer Etherspaltung unterwirft und/oder die so erhaltenen Verbindungen gegebenenfalls in ihre Salze mit physiologisch verträglichen Säuren überführt.

7. Arzneimittel, enthaltend eine Verbindung der Formel I gemäß Anspruch 1.

8. Verbindung gemäß Anspruch 1 zur Verwendung bei der Bekämpfung von Krankheiten.

**Claims**

1. An ω-cyano-1, ω-diphenyl-azaalkane derivative of the general formula I

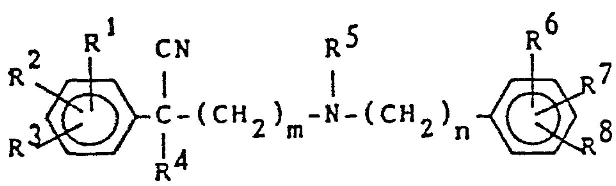

(I)

where

$R^1$, $R^2$, $R^3$, $R^6$, $R^7$ and $R^8$ are identical or different and each is hydrogen, halogen, hydroxyl, trifluoromethyl, $C_1$-$C_4$-alkyl, nitro, $C_1$-$C_4$-alkoxy or $C_1$-$C_4$-alkylmercapto, and two radicals in adjacent positions can together form a methylenedioxy, ethylenedioxy or 1,3-dioxatetramethylene group,

$R^4$ is straight-chain or branched, saturated or unsaturated alkyl of 9 to 20 carbon atoms,

$R^5$ is hydrogen or $C_1$-$C_4$-alkyl and

m and n are identical or different and each is from 2 to 4,

and salts thereof with a physiologically tolerated acid.

2. 1,7-bis-(3-Methoxyphenyl)-3-methylaza-7-cyano-nonadecane.

3. 1-(3-Methoxyphenyl)-3-methylaza-7-cyano-7-(3,4-dimethoxyphenyl)-nonadecane.

4. 1-(3-Methoxyphenyl)-3-methylaza-7-cyano-7-(3,4,5-trimethoxyphenyl)-nonadecane.

5. 1,7-Diphenyl-3-methylaza-7-cyano-nonadecane.

6. A process for the preparation of a compound of the formula I as claimed in claim 1, wherein

a) a phenylacetonitrile of the formula II

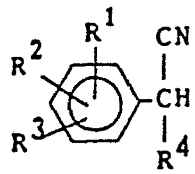

(II)

where $R^1$, $R^2$, $R^3$ and $R^4$ have the above meanings, is reacted with a 1-phenylazaalkane of the formula III

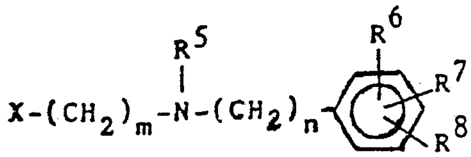

(III)

where $R^5$, $R^6$, $R^7$, $R^8$, m and n have the above meanings and X is a leaving group, or

b) an ω-cyano-1, ω-diphenyl-azaalkane derivative of the formula IV

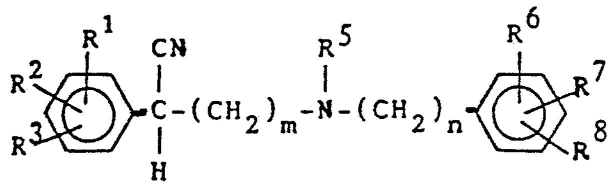

(IV)

where $R^1$, $R^2$, $R^3$, $R^5$, $R^6$, $R^7$, $R^8$, m and n have the above meanings, is reacted with a compound of the formula V

$$R^4—Y \qquad (V)$$

where $R^4$ has the above meanings, and Y is a leaving group, or

c) a phenylacetonitrile of the formula VI

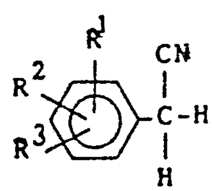

(VI)

where $R^1$, $R^2$ and $R^3$ have the above meanings, is reacted with a 1-phenylazaalkane of the formula III and a compound of the formula V, or

d) a phenylacetonitrile of the formula VII

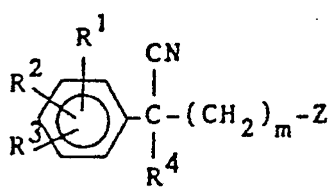

(VII)

where $R^1$, $R^2$, $R^3$, $R^4$ and m have the above meanings and Z is a leaving group, is reacted with a phenylalkylamine of the formula VIII

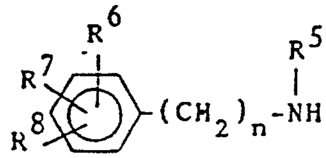
(VIII)

where $R^5$, $R^6$, $R^7$, $R^8$ and n have the above meanings, or

e) an ω-amino-alkyl-phenylacetonitrile of the formula IX

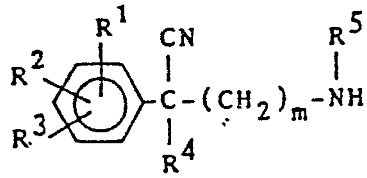
(IX)

where $R^1$, $R^2$, $R^3$, $R^4$, $R^5$ and m have the above meanings, is reacted with a phenylalkyl derivative of the formula X

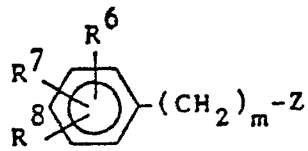
(X)

where $R^6$, $R^7$, $R^8$, m and Z have the above meanings, or

f) an ω-cyano-1, ω-diphenyl-azaalkane derivative of the formula XI

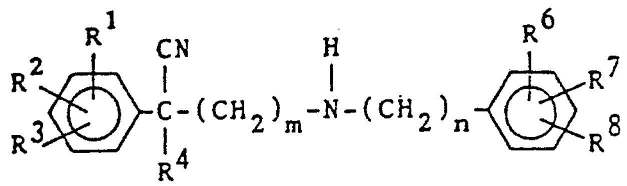
(XI)

where $R^1$, $R^2$, $R^3$, $R^4$, $R^6$, $R^7$, $R^8$, m and n have the above meanings, is alkylated, or

g) an ω-amino-alkyl-phenylacetonitrile of the formula IX is reacted with an aldehyde of the formula XII

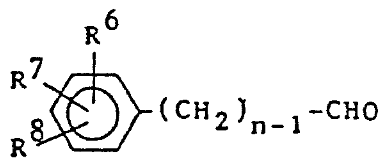
(XII)

where $R^6$, $R^7$, $R^8$ and n have the above meanings, under reducing conditions, or

h) an aldehyde of the formula XIII

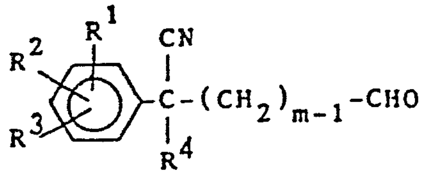
(XIII)

where $R^1$, $R^2$, $R^3$, $R^4$ and m have the above meanings, is reacted with a phenylalkylamine of the formula VIII under reducing conditions,

and, if desired and if one or more of $R^1$, $R^2$, $R^3$, $R^6$, $R^7$ and $R^8$ are alkoxy, the resulting compound is subjected to ether cleavage, and/or, if desired, the resulting compound is converted into a salt with a physiologically tolerated acid.

7. A drug containing a compound of the formula I as claimed in claim 1.

8. A compound as claimed in claim 1 for use in combating disorders.

**Revendications**

1. Dérivés de ω-cyano-1, ω-diphényl-aza-alcanes de la formule générale I

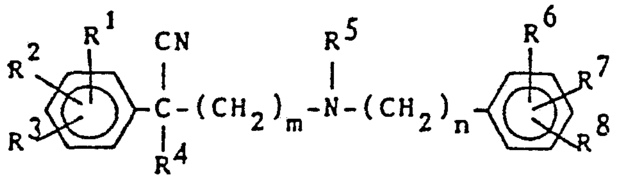

(I)

dans laquelle

$R^1$, $R^2$, $R^3$, $R^6$, $R^7$ et $R^8$, qui peuvent être identiques ou différents, désignent des atomes d'hydrogène ou d'halogène ou des groupes hydroxyle, trifluorométhyle, alkyle en $C_1$ à $C_4$, nitro, alcoxy en $C_1$ à $C_4$ ou alkyl (en $C_1$ à $C_4$)-mercapto, des paires de restes voisins pouvant former des groupes méthylène-dioxy, éthylène-dioxy ou 1,3-dioxa-tétraméthylène,

$R^4$ désigne un radical alkyle en $C_9$ à $C_{20}$ à chaîne droite ou ramifiée, saturé ou non,

$R^5$ désigne un atome d'hydrogène ou un radical alkyle en $C_1$ à $C_4$ et

m et n, qui peuvent être identiques ou différents, valent 2 à 4,

ainsi que leurs sels d'acides physiologiquement compatibles.

2. Le 1,7-bis-(3-méthoxyphényl)-3-méthyl-aza-7-cyano-nonadécane.
3. Le 1-(3-méthoxyphényl)-3-méthyl-aza-7-cyano-7-(3,4-diméthoxyphényl)-nonadécane.
4. Le 1-(3-méthoxyphényl)-3-méthyl-aza-7-cyano-7-(3,4,5-triméthoxyphényl)-nonadécane.
5. Le 1,7-diphényl-3-méthyl-aza-7-cyano-nonadécane.
6. Procédé de préparation des dérivés de la formule I selon la revendication 1, caractérisé en ce que :

a) on fait réagir des phényl-acétonitriles de la formule II

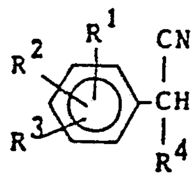

(II)

dans laquelle $R^1$ à $R^4$ possèdent la signification définie, et des 1-phényl-aza-alcanes de la formule III

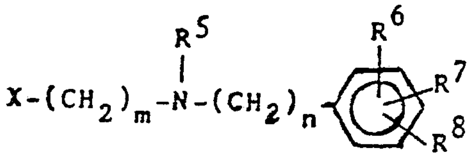

(III)

dans laquelle $R^5$ à $R^8$ et m et n possèdent la signification définie et X désigne un groupe clivable, ou

b) on fait réagir des dérivés d'ω-cyano-1, ω-diphényl-aza-alcanes de la formule IV

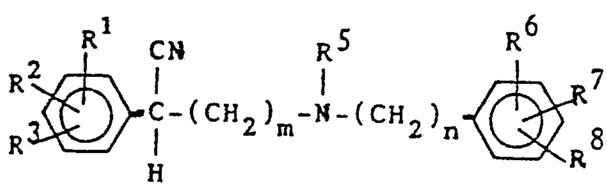

(IV)

dans laquelle $R^1$ à $R^8$ et m et n possèdent la signification définie, et des composés de la formule

$$R^4—Y \qquad (V)$$

dans laquelle $R^4$ possède la signification définie et Y représente un groupe clivable, ou

c) on fait réagir des phényl-acétonitriles de la formule VI

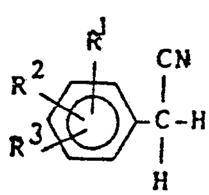

(VI)

dans laquelle $R^1$ à $R^3$ possèdent la signification définie, avec des 1-phényl-aza-alcanes de la formule III et des composés de la formule V, ou

d) on fait réagir des phényl-acétonitriles de la formule VII

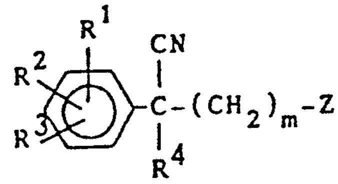

(VII)

dans laquelle $R^1$ à $R^4$ possèdent les significations définies et Z représente un groupe clivable, avec une phényl-alkyl-amine de la formule VIII

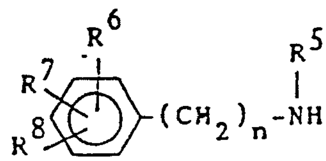

(VIII)

dans laquelle $R^5$ à $R^8$ possèdent la signification définie, ou

e) on fait réagir des ω-amino-alkyl-phényl-acétonitriles de la formule IX

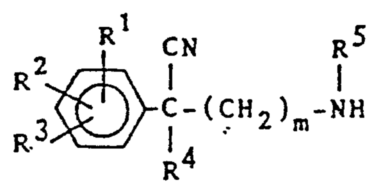

(IX)

dans laquelle $R^1$ à $R^5$ et n possèdent la signification définie, et des dérivés de phényl-alkyle de la formule X

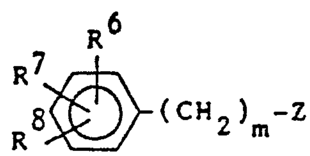

(X)

dans laquelle $R^6$ à $R^8$ et m et Z possèdent la signification définie, ou

f) on soumet à une alkylation des dérivés d'ω-cyano-1, ω-diphényl-aza-alcanes de la formule XI

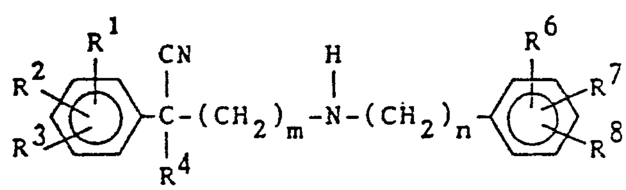

(XI)

dans laquelle $R^1$ à $R^8$ et m et n possèdent la signification définie, ou

g) on fait réagir des ω-amino-alkyl-phényl-acétonitriles de la formule IX, dans des conditions réductrices, avec des aldéhydes de la formule XII

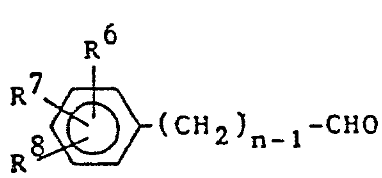

(XII)

dans laquelle $R^6$ à $R^8$ et n possèdent la signification définie plus haut, ou

h) on fait réagir des aldéhydes de la formule XIII

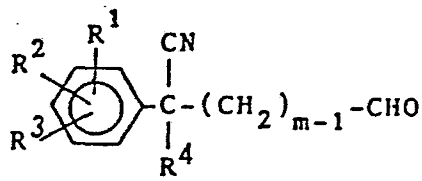

(XIII)

dans laquelle $R^1$ à $R^4$ et m possèdent la signification définie plus haut, dans des conditions réductrices, avec des phényl-alkyl-amines de la formule VIII,
les composés ainsi obtenus étant éventuellement soumis, lorsque un ou plusieurs des substituants $R^1$, $R^2$, $R^3$, $R^6$ $R^7$ et(ou) $R^8$ sont des radicaux alcoxy, à un clivage éthéré et(ou) transformés éventuellement en des sels d'acides physiologiquement acceptables.

7. Médicament, contenant un composé de la formule I selon la revendication 1.

8. Composé selon la revendication 1, destiné au traitement de maladies.